# EUROPÄISCHE PATENTSCHRIFT

(11) **EP 0 442 092 B1**
(45) Veröffentlichungstag und Bekanntmachung des Hinweises auf die Patenterteilung: **24.05.1995**
(21) Anmeldenummer: 90124369.1
(22) Anmeldetag: 17.12.1990
(51) Int. Cl.: B29C 53/64, B29C 70/00

(54) **Verfahren zur Herstellung von Faserverbund-Bauteilen**
Method of manufacturing fibre reinforced products
Procédé pour la fabrication de produits renforcés de fibres

(30) Priorität: 14.02.1990 DE 4004473
(43) Veröffentlichungstag der Anmeldung: 21.08.1991
(73) Patentinhaber: MAN Ceramics GmbH, 94453 Deggendorf (DE)
(72) Erfinder: Dierl, Rudolf, W-8060 Dachau (DE)

(56) Entgegenhaltungen:
- EP-A- 0 321 228
- WO-A-87/04916
- FR-A- 2 620 375

## Beschreibung

Die Erfindung bezieht sich auf ein Verfahren zur Herstellung von Bauteilen mit mindestens einer Lage aus längs des Bauteiles gerichteten unidirektionalen Fasern, unter Anwendung einer Flechtmaschine, bei dem ein vorgefertigter Stützkern zusammen mit das Bauteil zumindest teilweise umgebenden, unter Spannung gehaltenen unidirektionalen Fasern und Flechtfasern durch ein Flechtauge geführt werden, wobei zumindest ein Teil der Flechtfasern ein Flechtwerk bilden, das die unidirektionale Faserlage umgibt und die Fasern auf das Bauteil aufgelegt und mittels Harz verklebt werden.

Aus der DE 34 13 601 A1 ist ein Verfahren bekannt, bei dem zur Herstellung von Verbundprofilen ein vorgefertigter Stützkern zusammen mit umgebenden Verstärkungsfasern in einen Formkanal eingeführt, mit flüssigem Kunstharz getränkt und anschließend einem Aushärtungsprozeß unterworfen wird. Hierdurch können Bauteile mit längs des Bauteiles gerichteten, sogenannten unidirektionalen Fasern fertigungstechnisch einfach umgeben werden. Allerdings ist das Verfahren nur bei Bauteilen geeignet, die einen konstanten Querschnitt haben.

Aus der FR-A-2 620 375 ist ein Verfahren der eingangs genannten Art bekannt, mit dem die vorstehenden Probleme gelöst werden und Bauteile jeder Konfiguration unter Beibehaltung der fertigungstechnischen Einfachheit mit unidirektionalen Fasern überzogen werden können.

Zur Herstellung von Knochen-Implantaten, die in der Regel unregelmäßige Konturen haben, ist ein Verfahren aus der DE 40 04 474 bekannt, bei dem als Fadenauge eine Membran verwendet wird, deren Öffnung sich automatisch an die Kontur des durchzuführenden Bauteiles anpaßt.

Bei Anwendung dieser Methode können sich jedoch noch Probleme mit gekrümmten Bauteilen, wie z. B. der Schaft eines Hüftgelenkes, ergeben. Die den Kern umgebenden, unter Spannung gehaltenen Fasern werden bei Krümmungen des Stützkernes den kürzeren Weg einnehmen wollen, wodurch ein Verrutschen der Faser zur konkaven Seite des Bauteiles sowie das Abheben der Fasern an konkaven Seiten möglich ist.

Der Erfindung liegt die Aufgabe zugrunde, ein Verfahren zu schaffen, mit dem die Herstellung auch von gekrümmten Knochen-Implantaten möglich ist.

Die Aufgabe wird erfindungsgemäß mit den Merkmalen des Anspruches 1 gelöst.

Die Fasern des Flechtwerkes winden sich automatisch um den Kern herum und drücken dabei zwischen Kern und Flechtfasern befindliche unidirektionale Fasern an den Kern. Hierbei ersetzen die Flechtfasern bei der Herstellung von Implantaten nicht nur die bei dem bekannten Verfahren verwendete Membran, sondern sie bilden gleichzeitig einen Torsionskasten für das Implantat, der die bei Belastung auftretenden Radialkräfte aufnimmt.

Aufgrund der Krümmung, die ein Implantatschaft aufweist, kann zumindest ein Teil der unidirektionalen Fasern umflochten werden, um diese entsprechend in der Krümmung führen zu können.

In diesem Fall werden die gewählten unidirektionalen Fasern mit Flechtfasern unterlegt, so daß diese Fasern in ihrer Wirkung an Festigkeit einbüßen. Ein Ausgleich kann allerdings durch Anhebung der Faserlagenzahl erreicht werden. Es ist aber auch möglich, durch Wahl sehr dünner Flechtfasern die Festigkeitseinbußen zu verringern.

Es ist selbstverständlich jede Kombination möglich, bei der mehr oder weniger unidirektionale Fasern zwischen dem Kern und den Flechtfasern bzw. innerhalb des Geflechtes geführt werden.

Die Einbringung eines Harzes erfolgtnach konventionellen Methoden, z. B. durch Tränken der Fasern vor oder nach deren Aufbringung auf den Stützkern. Das nach dem oben beschriebenen Verfahren hergestellte Bauteil wird anschließend oder während des Durchziehens durch das Fadenauge einer Wärmebehandlung unterzogen, um das Harz auszuhärten.

Die Enden des Schaftes können ausschließlich aus den unidirektionalen Fasern und den Flechtfasern bestehen, wobei der Stützkern lediglich als Verdrängerkörper zur Bildung des Bereiches mit dickerem Querschnitt verwendet wird.

Zur Anwendung des erfindungsgemäßen Verfahrens kann eine im Handel erhältliche Flechtmaschine verwendet werden, die um Faserführungen für die unidirektionalen Fasern erweitert wird.

Die Erfindung wird anhand von in der Zeichnung schematisch dargestellten Ausführungsbeispielen näher beschrieben.
Es zeigen:
- Fig. 1 und 2: eine Anlage zum Durchführen des Verfahrens im Schnitt bzw. in Draufsicht,
- Fig. 3: den Faserverlauf an einem Bauteil und
- Fig. 4 und 5: je einen Querschnitt eines fertigen Bauteiles.

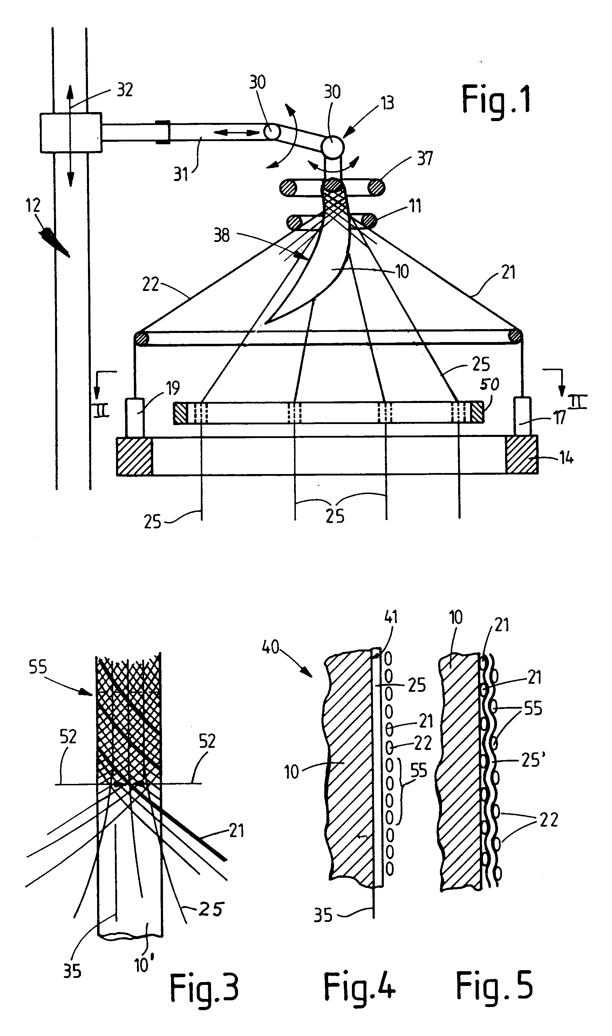

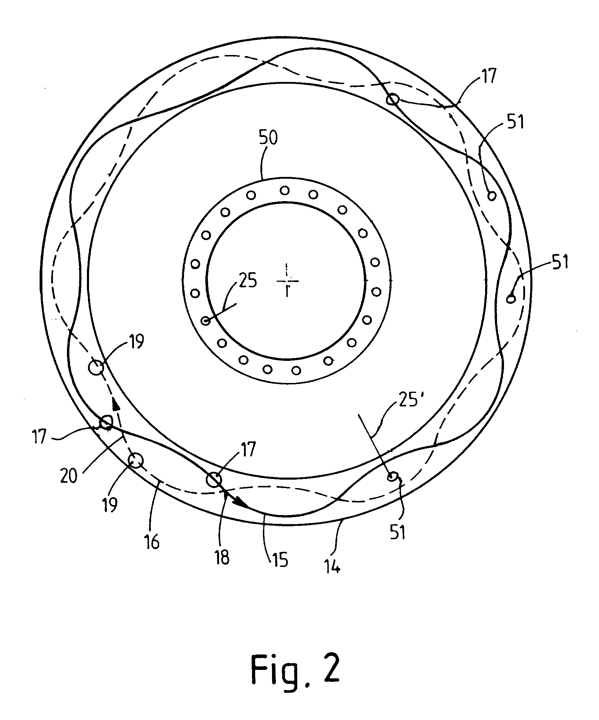

In Fig. 1 ist die Herstellung eines Prothesenschaftes gezeigt, der zunächst aus einem vorgefertigten Stützkern 10 besteht. Der Stützkern 10 wird durch ein Fadenauge 11 einer Flechtmaschine 12 mittels eines Greifers 13 durchgezogen. Die Flechtmaschine 12 besteht aus einem Führungsring 14, auf dem zwei sinusförmige Führungsschienen 15 bzw. 16 (Fig. 2) vorgesehen sind. In der einen Schiene 15 wird eine bestimmte Anzahl von Klöppel 17 einer ersten Gruppe in eine Richtung 18 geführt, während eine zweite Gruppe Klöppel 19 auf der zweiten Schiene 16 in die Gegenrichtung (20) geführt wird.

Bei der so beschriebenen Bewegung der Klöppel 17 bzw. 19 wird jede einzelne Faser 21 einmal unter eine andere Faser und einmal über eine andere Faser gelegt. Zusätzlich zu den Flechtfasern 21, 22 der beiden Klöppelgruppen 17, 19 sind über einen inneren Ring 50 (Fig. 2) geführte unidirektionale Fasern 25 vorgesehen, die zwischen dem Kern 10 und den Flechtfasern 21, 22 durch das Fadenauge 11 durchgezogen werden. Sämtliche Faserenden werden mit dem Greifer 13 verbunden, der gleichzeitig den Stützkern 10 trägt und ihn mit einer vorgegebenen Geschwindigkeit vom Fadenauge 11 wegbewegt. Für gekrümmte oder unregelmäßige Bauteile, wie es beim Prothesenschaft der Fall ist, ist der Greifer 13 mit Gelenken 30, Teleskopeinrichtung 31 oder dergleichen ausgerüstet, um zusätzlich zur Vorschubbewegung 32 auch noch Schwenkbewegungen durchführen zu können. Damit kann das Bauteil jeweils so gerichtet werden, daß die Achse des Bauteilbereiches, das gerade auf der Fadenaugenebene liegt, auf der Flechtmaschinenachse steht. Auf diese Weise werden die unter Spannung gehaltenen unidirektionalen Fasern 25 stets den Mantellinien 35 (Fig. 3) des Bauteiles folgen.

Die einzelnen Flechtfasern 21 bzw. 22 beschreiben im Verlauf des Flechtprozesses eine Schraubenlinie, wie sie in Fig. 3 mit der dickgezeichneten Faser 21 gezeigt ist. Bei diesem Verlauf drückt die Flechtfaser 21 die unidirektionalen Fasern, die der Mantellinie 35 des Stützkernes 10' folgen, an den Stützkern 10 bzw. 10'. Die Steigung der umwickelten Flechtfasern bzw. deren Orientierung am fertigen Bauteil wird durch die geometrischen Verhältnisse der Flechtmaschine 12, dem Querschnitt des Bauteiles bzw. Stützkernes 10 und der Geschwindigkeit, mit der das Bauteil 10 bewegt wird, bestimmt. Durch Automatisierung und Programmierung des Greiferbewegungsmechanismus kann die Vorschubgeschwindigkeit entsprechend der Geometrie bzw. dem Querschnitt des Bauteiles 10 verändert werden, um die gewünschte Orientierung der Flechtfasern 21, 22 über das gesamte Bauteil zu erhalten.

Sowohl die Flechtfasern 21 als auch die unidirektionalen Fasern 25 können vorab durch Tränkeinrichtungen mit einer Matrix getränkt werden. Die Aushärtung des Harzes wird mittels der über dem Fadenauge 11 vorgesehenen Heizeinrichtung 37 bewirkt. Die Einführung des Matrixmaterials sowie deren Härtung kann auch auf jede andere bekannte Art durchgeführt werden.

Bei den bisher beschriebenen Schritten bilden die unidirektionalen Fasern 25 eine innere Faserschicht, die von dem Flechtwerk 55 umgeben ist. In einem Längsschnitt durch das fertige Bauteil 40, wie in Fig. 4 gezeigt, ist an der Oberfläche 41 des Stützkernes 10 eine Längsfaser 25 zu sehen, auf der sich die Flechtfasern 21 bzw. 22 befinden. In diesem Fall legt sich die unidirektionale Faser 25 genau parallel an die Mantellinie 35 des Stützkernes 10 an. Sie ist dabei gestreckt, wodurch das Bauteil die optimale Festigkeit für Druck/Zug-Belastungen hat.

Wenn ein Bauteil starke Krümmungen aufweist, wie es z. B. beim Prothesenschaft der Fall sein kann, wäre zusätzlich zur radialen Führung der unidirektionalen Fasern 25 eine tangentiale Führung zweckmäßig, um ein Abrutschen der unter Zugspannung stehenden Fasern 25 in den Krümmungsbereichen 38 des Stützkernes 10 zu vermeiden. In diesem Fall wird ein Teil der unidirektionalen Fasern 25 nicht innerhalb des, sondern durch den Flechtring 14 geführt. Dieses ist in Fig. 2 näher gezeigt, bei dem der innere Ring 50 nicht alle, wie oben beschrieben, sondern nur einen Großteil der unidirektionalen Fasern 25 führt, während im Flechtring 14 stehende Fadenaugen 51 für unidirektionale Fasern 25' vorgesehen sind. Diese Fasern 25' werden abwechselnd von der einen Klöppelgruppe 17 unterfahren und von der anderen Klöppelgruppe 19 überfahren, so daß diese unidirektionalen Fasern 25' zusätzlich zur radialen Führung durch die Flechtfasern 21, 22 auch eine tangentiale Führung, wie in Fig. 3 mit den Pfeilen 52 angedeutet, erfahren. Damit werden sie in ihrer Position, d. h. entlang der entsprechenden Mantellinie 35 des Stützkernes 10' gehalten.

In diesem Fall wird, wie in Fig. 5 gezeigt, die entsprechende unidirektionale Faser 25' stellenweise durch Flechtfasern 21 unterlegt und dazwischen mit Flechtfasern 22 umwickelt, so daß die unidirektionale Faser 25' ihre optimale gespannte Lage verliert. Mit sehr dünnen Flechtfasern 21 bzw. 22 kann dieser Nachteil minimiert werden, wenn es darauf ankommt. Das Verhältnis der durch den inneren Ring 50 und dem Flechtring 14 geführten unidirektionalen Fasern 25 bzw. 25' kann beliebig gewählt werden. Es wird sich in der Regel nach dem jeweiligen Anwendungsfall und dessen Anforderungen richten.

Für die Herstellung eines Schaftes für ein Hüftgelenk-Implantat mit unidirektionalen Fasern wird man das Verhältnis zwischen im Innenring 50 und im Flechtring 14 geführten unidirektionalen Fasern 25 bzw. 25' möglichst groß wählen.

## Patentansprüche

1. Verfahren zur Herstellung von Bauteilen mit mindestens einer Lage aus längs des Bauteiles gerichteten unidirektionalen Fasern unter Anwendung einer Flechtmaschine, bei dem ein vorgefertigter Stützkern zusammen mit das Bauteil zumindest teilweise umgebenden, unter Spannung gehaltenen, unidirektionalen Fasern und Flechtfasern (21, 22) durch ein Flechtauge (11) geführt werden, wobei zumindest ein Teil der Flechtfasern ein Flechtwerk (55) bilden, das die unidirektionale Faserlage (25) umgibt und wobei die Fasern auf das Bauteil aufgelegt und mittels Harz verklebt werden, gekennzeichnet durch Anwendung des Verfahrens zur Herstellung von Knochenimplantaten.

2. Verfahren nach Anspruch 1, dadurch gekennzeichnet, daß die unidirektionalen Fasern (25) zwischen dem Stützkern (10,10') und den Flechtfasern (21, 22) geführt werden.

3. Verfahren nach Anspruch 1 oder 2, dadurch gekennzeichnet, daß die unidirektionalen Fasern (25) zumindest teilweise auf der Ebene der Flechtfasern (21, 22) geführt werden, derart, daß sie umflochten werden.

## Claims

1. A process for manufacturing components with at least one layer of unidirectional fibres oriented along the length of the component, using a braiding machine, in which process a pre-manufactured supporting core, together with braiding fibres (21, 22) and unidirectional fibres under tension and at least partially surrounding the component, are fed through a braiding eye (11), wherein at least a part of the braiding fibres form an interlaced work (55) surrounding the unidirectional fibre layer (25), and the fibres are laid on the component and stuck in place using resin, **characterised by** use of the process to manufacture bone implants.

2. A process in accordance with claim 1, **characterised in that** the unidirectional fibres (25) are guided between the supporting core (10, 10') and the braiding fibres (21, 22).

3. A process in accordance with claim 1 or 2, **characterised in that** the unidirectional fibres (25) are fed at least partially in the plane of the braiding fibres (21, 22) in such a manner that braiding takes place round the said unidirectional fibres.

## Revendications

1. Procédé pour fabriquer des pièces ayant au moins une couche de fibres unidirectionnelles dirigées longitudinalement par rapport à la pièce, en utilisant une machine à tresser, selon lequel un noyau d'appui préfabriqué ainsi que des fibres unidirectionnelles maintenues sous tension et des fibres tressées (21, 22) entourant partiellement la pièce, est conduit à travers un oeillet de tressage (11), au moins une partie des fibres de tressage formant un tressage (55) qui entoure la couche de fibres (25) unidirectionnelles, puis les fibres sont appliquées sur la pièce et collées avec de la résine, procédé caractérisé en ce qu'on l'applique à la fabrication d'implants osseux.

2. Procédé selon la revendication 1, caractérisé en ce que les fibres unidirectionnelles (25) sont guidées entre le noyau d'appui (10, 10') et les fibres tressées (21, 22).

3. Procédé selon la revendication 1 ou 2, caractérisé en ce que les fibres unidirectionnelles (25) sont guidées au moins partiellement dans le plan des fibres tressées (21, 22) pour être intégrées dans le tressage.
